# EUROPEAN PATENT APPLICATION

(11) **EP 1 944 360 A1**
(43) Date of publication of application: **16.07.2008**
(21) Application number: 08000517.6
(22) Date of filing: 11.01.2008
(51) Int. Cl.: C12N 5/00, C12N 5/06, A61K 39/00

(54) **Method for culturing dendritic cells (DC) and cytokine-induced killer cells (D-CIK) and applications thereof**

(30) Priority: 12.01.2007 TW 96101280
(71) Applicant: Koyo International Holding Corporation, Songshan District Taipei City 105 (TW)
(72) Inventor: Xia, Jian-Chuan, Guangzhou (CN)
(74) Representative: Arth, Hans-Lothar

(57) **Abstract**

A mass production of dendritic cells (DC) and cytokine-induced killer cells (D-CIK) cells from the umbilical cord blood or peripheral blood via the activation with a cellular activator "Koyo" derived from the purified tumor cells during the aggregation and proliferation of the purified tumor cells is provided. A preparation of a vaccine with the dendritic cells (DC) and D-CIK cells is used to improve the symptoms of infectious diseases, cancers, metastasised cancers and auto-immune disorders.

## Description

### BACKGROUND OF THE INVENTION

### Field of Invention

The present invention relates to an area of tumor immunology More particularly, the present invention relates to an application of a method for growing and proliferating highly purified tumor cells to derive cellular activators (Koyo) for activating dendritic cells (DC) or D-cytokine induced killer cells (D-CIK) from umbilical cord blood or peripheral blood, wherein the activated cells are infused back into the patents for medical treatments and for researches in infection, abnormal proliferation and auto-immune disorders.

### Description of Related Art

The functions of antigen-presenting cells (APCs) are to internalize and process antigen, and then display a fragment of the antigen on their membrane to the T lymphocytes (T cells). The T cells are stimulated and interact with the antigen on the membrane of the antigen presenting cell by proliferating into effector cells and generating immunological response to infections and even cancer cells (Shimizu J. et al. 1991 J. Immunol. 146:1708-1714; Zou, J. et al. 1992 Cancer Immunol Immunolther. 35:1-6; Takahashi, H. et al. 1993 International Immunology 5:849-857). Pulsed APC are normally generated as follows : (i) many peptide fragments, for example, peptide fragments of the antigen are being guided to and displayed on the membrane of the antigen-presenting cells (Mehta-damani, A. et. al., J. Immunol. 153 : 996-1003, 1994) ; or (ii) whole protein or protein particles that being cultured together with APC are absorbed by APC and then processed into small peptide fragments which are being displayed on the membrane of the antigen-presenting cells (Cohen, P.A. et al., Cancer Res. 54 : 1055-1058, 1994).

T lymphocytes (for example, T cells) are different from B lymphocytes (for example B cells), wherein T lymphocytes can only recognize the specific antigen peptide on the surface of APC, for example, the antigen-MHC (major histocompatibility complex) molecules, in which T cells only recognize antigens presented by the MHC molecules. This phenomenon is known as MHC restriction. The APC cells generally express MHC molecules, stimulate T helper cells as well as cytotoxic T lymphocytes (CLT) and present antigen to the T cells via the MHC molecules.

Dendritic cells (DC) have the broadest range of antigen presentation, and are probably the most important APC. At certain development stages, DC grow branched projections, the dentrites. Dentritic cells can significantly stimulate the proliferation of native T cell, while other APC, such as MΦ or B cells, can only stimulate the activated or memory T cells. Accordingly, dendritic cells play an important role as the initiator to induce mechanical immune response.

The human DC cells can be generated from CD34+ cells and cell factors. The generation of human DC cells can be achieved by simulating granulocyte-macrophage colony-stimulating factor (GM-CSF) from the mono-nucleus CD34 and interleukin-4 (IL-4) to differentiate into immature DC cells. These immature DC cells are further differentiated into mature cells under the regulations of tumor necrosis factor-α (TNF-α) or CD 40 ligand (CD40L). The membrane of the mature DC cells includes cell factors such as MHC class II, MHC class I, co-stimulator CD80, CD86, intercellular adhesion molecule (ICAM), IL-12, TNF-α, interferon-γ (IFN-γ), etc., for participating in immune regulation.

According to the sources of DC cells, there are two major categories of the DC cells: myeloid DC and lymphoid DC. A majority of the DC cells are originated from the bone marrow and the DC cells are circulated from the bone marrow to the peripheral blood and then distributed to tissues of the entire body. Although DC cells are fully distributed throughout the entire body, their concentration is less than 1% of the mono-nucleus cells in the peripheral blood. According to the different distributed regions, DC can further divide into DC cells in lymphocytes and DC cells in fluid. Normally, DC cells are preserved under the immature state in which the expression levels of co-stimulation factor and adhesion factor are low and the in vitro stimulation of the mixed leukocyte reaction (MLR) is weak. However, the DC cells under the immature state are characterized by high endocytic or phagocytic activity of antigens and process capability (or degrade) of the antigen protens into small pieces. Once they come in contact with the antigens or being suject to simulation, for example, by lipopolysaccharide (LPS), IL-1, TNF-α, the dendritic cells become mature cells. The characteristics of the mature cells include the increased expression of MHC Class I, MHC Class II, CD 80 (B7-1), CD86 (B7-2), CD 40, intercellular adhesion molecule -1 (ICAM-1) on the membrane. The conversion of immature to mature dendritic cells is accompanied by a marked cellular reorganization, including the redistribution and the increased expression of major histocompatibility complex class I molecules (MHC I), major histocompatibility complex class II molecules (MHC II), CD 80 (B7-1), CD86 (B7-2), CD 40, on the membrane. Further, the capability of stiminulating mixed lymphocyte reaction is strong, while the endocytic and processing activities of pathogens or antigens are greatly reduced. During the maturation of the dendritic cells (activated), these cells migrate from the peripheral tissues (received the antigen signal) to the lymphoid tissues via blood and/or lymph where they interact with T cells to initiate the immune response. The maturation of the DC cells derived from the stem cells in the bone marrow undergoes four stages of phenotypical and functional changes: primary stage, pre-mature stage, migration stage and mature stage.

Following the antigen uptake and processing into small antigenic peptide fragments, the antigenic peptides are then transferred to the secondary lymphoid organs and then be presented via MHC molecules on the DC surface to T cells. T-cells are activated to initiate the immune response. Concurrently, the DC cells up-regulates cell-surface that act as co-receptors in T-cell activation, such as CD 80 and CD86, to enhance the activation of T cells, the activation of helper T-cells and CTL cells as well as B-cells.

DC cells are essential in the treatment of bacterial and viral infections. DC cells are particular important in the applications of treatment of patients having serious infections, vaccine and immune therapy. For example, a specific cancer immune therapy involves in the application of in vitro cultured dendritic cells carrying specific tumor antigens, wherein the dentritic cells are infused into the patient in a form of a vaccine in order to stimulate specific immune reaction for cancer treatment. The feasibility of in vitro cultured dendritic cells on specific cancer therapy has been confirmed in animal models.

However, the isolation and recognition of the DC cells remain a challenge due to their reactions with soluble factors and certain unknown mechanisms. Accordingly, their regulation, development and physiology are difficult to control in medical application.

Cytokine-induced killer cells (CIK) can be cultured in vitro with human mono-nucleus cells, originally collected from the peripheral blood, and several cell factors, for a period of time. CIK cells can express CD+3 and CD+56, two types of membrane proteins at the same time, and also known as the NK (natural killer)-T cells, in which both the anti-tumor activity of T lymphocytes and the non-MHC restriction anti-tumor effect are preserved. Accordingly, CIK has become an appropriate candidate for a new generation of anti-tumor, known as an adoptive immune cell therapy. However, clinical reports, especially on the application of malignant tumor, are limited.

A cellular activator (Koyo) extracted from a primary tumor cell culture can be used to culture dendritic cells. The therapeutic effect of the DC stimulated CIK cells (D-CIK) in clinical application is critical in the research of tumor cell antigen and drug developments in the fields of anti-tumor and anti-infections.

### SUMMARY OF THE INVENTION

In view of the foregoing, the present invention provides a method for purifying undamaged tumor cells in an accelerated and convenient manner, and the cellular activator (Koyo) derived from the differentiation of the live tumor cells are obtained to activate dendritic cells and D-CIK cells.

The present invention also provides an application of cellular activator (Koyo) to activate dendritic cells or D-CIK cells obtained from umbilical blood or peripheral blood of a patient, wherein the activated dendritic cells or D-CIK cells can be used to fabricate vaccine or anti-tumor effector cells to enhance immunity.

The present invention further provides an application of cellular activator to activate dendritic cells or D-CIK cells obtained from umbilical blood or peripheral blood of a patient and the activated dendritic cells or D-CIK cells can be used to fabricate vaccine or anti-tumor effector cells to improve symptoms of patients with viral infections.

The present invention further provides an application of cellular activator to activate dendritic cells or D-CIK cells obtained from umbilical blood or peripheral blood of a patient, wherein the activated dendritic cells or D-CIK cells can be used to fabricate vaccine or anti-tumor effector cells to improve symptoms of patients with cancers or metastasized cancers.

The present invention further provides an application of cellular activator to activate dendritic cells or D-CIK cells obtained from umbilical blood or peripheral blood of a patient, wherein the activated dendritic cells or D-CIK cells can be used to fabricate vaccine or anti-tumor effector cells to improve symptoms of patients with auto-immune disorders.

The present invention provides a convenient and accelerated method to remove non-tumor cells and necrotic cells and to isolate a mass quantity of highly purified and live tumor cells. Hence, the success rate of culturing primary tumor cells is enhanced. The application of this type of purification technique can be used for an effective culturing of cellular activators.

The present invention further provides a method using cellular activator derived from purified tumor cells to activate dendritic cells or D-CIK cells obtained from umbilical blood or peripheral blood of a patient, wherein the activated dendritic cells or D-CIK cells can be used to fabricate vaccine in which the dendritic cells or D-CIK cells are infused back to the patient to successfully improve the conditions of patients with the final stage of kidney cancer.

The present invention provides a method for activating dendritic cells, D-CIK cells (DC stimulated CIK cells) with cellular activator derived from purified human tumor cells obtained by the application of an isolation and purification technique.

According to an embodiment of the invention, in the above method for activating dendritic cells and D-CIK cells (DC stimulated CIK cells) with cellular activator derived by applying an isolation and purification technique on human tumor cells, the isolation purification technique on tumor cells includes (i) a pre- purifying treatment of tumor cells and (ii) isolation and purification of the tumor cells.

According to the above method for activating dendritic cells and D-CIK cells (DC stimulated CIK cells) with cellular activator cultured by the application of an isolation and purification technique on human tumor cells, the pre-purifying treatment of tumor cells includes examining and selecting the tumor tissues. The tumor tissues are then cut into pieces after being wet with a small amount of serum-free culturing medium RPMI-1640 and the pieces of tumor tissues are placed in first centrifuge tubes. Collagenase solution is further added and the centrifuge tubes are placed in a water bath. Diluting with a serum free culture medium RPMI-1640, the cell suspension is collected by pipetting repeatedly The suspension is then centrifuged to remove the upper layer of serum. An appropriate amount of 10% fetal calf serum or human AB serum and 1% double-antibody RPMI-1640 culturing medium are added and evenly mixed with the post-centrifuged cells.

According to an embodiment of the invention for activating dendritic cells, D-CIK cells (DC stimulated CIK cells) with cellular activator derived by the application of an isolation and purification technique on human tumor cells, the isolation and purification of tumor cells include obtaining an appropriate amount of fetal calf serum or human AB serum, centrifuging the serum and placing the supernatant in the second centrifuge tubes while placing the serum in the third centrifuge tubes. Placing an appropriate amount of the cell suspension liquid of the evenly mixed tumor tissues from the first centrifuge tubes into the third centrifuge tube along the sidewall of the third centrifuge tube. After allowing the solution in the third centrifuge tube remained undisturbed for a period of time, removing a portion of the serum from the bottom of the third centrifuge tube to place in the four centrifuge tube. After repeating the centrifugation process for several times, the precipitated cells are purified tumor cells.

According to the above method for activating dendritic cells and D-CIK cells (DC stimulated CIK cells) with cellular activator cultured by the application of an isolation purification technique on human tumor cells, the tumor cells include cells selected from the group consisting of parotid cancer, mediastinum transferred squamous cell carcinoma, melanoma, pancreatic cancer, thyroid gland cancer, duodenum papilloma cancer, prostate cancer, ovarian cancer, kidney cancer, lung cancer, breast cancer, nasopharyngeal carcinoma, cerebellum medulloblastoma, colon cancer, liver cancer, malignant glioma, rectal cancer, esophageal cancer and lung cancer.

The present invention provides an application of the above method for activating dendritic cells and D-CIK cells (DC stimulated CIK cells) with cellular activator cultured by the application of an isolation purification technique on human tumor cells to fabricate a vaccine for improving symptoms of infections.

According to the above vaccine for improving symptoms of infections of the present invention, the primary dendritic cells, D-CIK cells are obtained from umbilical cord blood or periphery blood of a patient.

According to the above vaccine for improving symptoms of infections of the present invention, the infections are resulted from antigen, virus, bacteria, parasites or fungus.

According to the above vaccine for improving symptoms of viral infections of the present invention, the types of virus include piconaviridae, caliciviridae, togaviridiae, flaviviridae, coronavirida, rhabdoviridae, filovirida, paramyxoviridae, orthomyxoviridae ; bunyaviridae, arenaviridae, reoviridae, retroviridae, hepadnaviridae, parvoviridae, papovaviridae, adenoviridae, herpersviridae and poxyviridae.

According to the above vaccine for improving symptoms of bacteria infections of the present invention, the types of bacteria are selected from the group consisting of P. aeruginosa, E. coli, Klebsiella species, Serratia, Pseudomonas, P. cepacia, Acinetobacter, S. epidermidis, E. facecalis, S. pneumoniae, S. aureus, Haemophilus, Neisseria, N. meningitides, Bacterioides, Citrobacter, Branhamella, Salmonella, Shigella species, S. pyrogenes species, Proteus species, Clostridium, Erysipelothrix species, Listeria species, Pasteurella multocida, Streptobacillus species, Spirillum species, Fusospirocheta species, Treponema palladium species, Borrelia species, Actinomycetes, Mycoplasma species, Chlamydia species, Rickettsia species, Spirochaeta, Legionella species, Mycobacteria species, Ureaplasma species, Streptomyces species, Trichomonas species and P. mirabilis.

According to the vaccine for improving symptoms of parasite infections of the present invention, the parasites are selected from the group consisting of Plasmodium falciparum, P. vicax, P. ovale, P. malaria, Toxoplasma gondii, Leishmania mexicana, L. tropica, L. major, L. aethiopica, L. donovanii, L. Trypanosoma cruzi, T. brucei, Schistosoma mansoni, S. haematobium, S. japonium, Trichinella spiralis, Wuchereria bancrofti, Brugia malayi, Entomoeba histolytica, Enterobius vermicularis, Taenia solium, T, saginata, Trichomonas vaginatis, T. hominis, T. tenax, Giardia lamblia, Cryptosporridium parvum, Pneumocytis carinii, Babesia bovis, B. divergens, B. microti, Isospora belli, Ascaris lumbricoides, necator americanis, Ancylostoma duodenale, Stronglyoides stercoralis, Capillaria philippinensis, Angiostrongylus cantonesis, Hymenolepis nana, Diphyllobothrium latum, Echinococcus granulosus, E. multilocularis, Paragoniumus westermani, Clonorchis sinensis, Ophisthorchisfelineus, O. viverrini, Fasciola hepatica, Sarcoptes scabiei, Pediculus humanus, Phthirus pubis and Dermatobia hominis.

According to the above vaccine for improving symptoms of fungus infections of the present invention, the fungus are selected from the group consisting of Cryptococcus neoformans, Blastomyces dermatitidis, Ajellomyces dermatitidis, Histoplasma capsulatum, Coccidioides immitis, Candida species, Aspergillus species, Rhizomucor species, Cunninghamella species, Apophysomyces species, Sporothrix schenkii, Paracoccidioides brasiliensis, Paseudallescheria boydii, Torulopsis glabrata, Dermatophytes species.

According to the above vaccine for improving symptoms of infections of the present invention, wherein the Candida species is elected from C. albicans, C. tropicalis, C. parapsilosis, C. guillermondii and C. krusei; the Aspergillus species is selected from A. fumigatus, A. flavus, and A. niger; and the Apophysomyces speces is selected from A. saksenaea, A. mucor and A. absidia.

According to the above vaccine for improving symptoms of infections of the present invention, the antigen is selected from the group consisting of Vibrio cholerae, enterotoxigenic Escherichia coli, rotavirus, Clostridium difficle, Shigella, Salmonella typhi, Parainfluenza virus, influenza virus, Streptococcus mutans, Plasmodium falciparum, Staphyloccus aureus, rabies virus and Epstein-Barr virus.

The present invention provides an application of the above method for activating dendritic cells and D-CIK cells (DC stimulated CIK cells) with cellular activator cultured by applying an isolation and purification technique on human tumor cells to fabricate a vaccine for improving symptoms of cancers and metastatic cancers.

According to the above vaccine for improving symptoms of cancers and metastatic cancers of the present invention, the primary dendritic cells, D-CIK cells are obtained from umbilical cord blood or periphery blood of a patient.

According to the above vaccine for improving symptoms of cancers and metastasized cancers of the present invention, the vaccine is applicable for improving symptoms of epithelial cancers include but not limited to parotid cancer, mediastinum transferred squamous cell carcinoma, melanoma, pancreatic cancer, thyroid gland papillomatous cystadenoma, duodenum papilloma cancer, prostate cancer, ovarian cancer, kidney cancer, lung cancer, breast cancer, nasopharyngeal carcinoma, cerebellum medulloblastoma, colon cancer, liver cancer, malignant glioma, rectal cancer, esophageal cancer, lung cancer, liver metastases from colon cancer and liver metastases from rectal cancer.

The present invention provides an application of the above method for activating dendritic cells and D-CIK cells (DC stimulated CIK cells) with cellular activator cultured by applying an isolation purification technique on human tumor cells to fabricate a vaccine for improving auto-immunity.

According to the above vaccine for improving auto-immunity of the present invention, the primary dendritic cells, D-CIK cells are obtained from umbilical cord blood or periphery blood of a patient.

According to the present invention, a fast, convenient, efficient and cost effective method is provided to isolate and purify tumor cells. Further, a mass quantity of the purified tumor cells can be obtained without inducing damages to the tumor cells to increase the success of the primary culture of tumor cells. The cell activator (Koyo) derived from the purified tumor cells can be applied for medical treatment and provided an alternative to chemotherapy and operation.

It is to be understood that both the foregoing general description and the following detailed description are exemplary, and are intended to provide further explanation of the invention as claimed.

### BRIEF DESCRIPTION OF THE DRAWINGS

The accompanying drawings are included to provide a further understanding of the invention, and are incorporated in and constitute a part of this specification. The drawings illustrate embodiments of the invention and, together with the description, serve to explain the principles of the invention.

Figure 1 illustrates an electron microscope photograph of human kidney cancer cells before purification.

Figure 2 illustrates an electron microscope photograph of human kidney cancer cells after purification.

Figure 3 illustrates the MR image of a 34-year-old male patient suffered from 18234 parotid cancer in example 3 before the treatment.

Figure 4 illustrates the MR image of a 34-year-old male patient suffered from parotid cancer in example 3 after the treatment.

Figure 5 illustrates the MR image of a 52-year-old male patient suffered from liver cancer in example 4 before the treatment.

Figure 6 illustrates the MR image of a 52-year-old male patient suffered from liver cancer in example 4 after the treatment.

Figure 7 illustrates the MR image of a 42-year-old male patient suffered from liver cancer in example 5 before the treatment.

Figure 8 illustrates the MR image of a 55-year-old male patient suffered from primary liver cancer in example 6 before the treatment.

Figure 9 illustrates the MR image of a 55-year-old male patient suffered from primary liver cancer in example 6 after the treatment.

Figure 10 illustrates the MR image of a 72-year-old male patient suffered from rectal cancer with liver metastasis in example 7 before the treatment.

Figure 11 illustrates the MR image of a 72-year-old male patient suffered from rectal cancer with liver metastasis in example 7 after the treatment.

Figure 12 illustrates the MR image of a 71-year-old female patient suffered from rectal cancer with liver metastasis in example 8 before the treatment.

Figure 13 illustrates the MR image of a 71-year-old female patient suffered from rectal cancer with liver metastasis in example 8 during the treatment.

Figure 14 illustrates the MR image of a 71-year-old female patient suffered from rectal cancer with liver metastasis in example 8 after the treatment.

Figure 15 illustrates the MR image of a 71-year-old male patient suffered from well differentiated lung cancer in example 9 after the treatment.

Figure 16 illustrates the MR image of a 63-year-old female patient suffered from well differentiated lung cancer in example 10 before the treatment.

Figure 17 illustrates the MR image of a 63-year-old female patient suffered from well differentiated lung cancer in example 10 after the treatment.

Figure 18 illustrates the MR image of a 63 year-old-male patient suffered from moderately differentiated lung cancer with bronchioloalveolar carcinoma in example 11 before the treatment.

Figure 19 illustrates the MR image of a 56-year-old female patient suffered from malignant melanoma at the heel in example 18 before the treatment.

Figure 20 illustrates the MR image of a 56-year-old female patient suffered from malignant melanoma at the heel in example 18 after the treatment.

Figure 21 illustrates the MR image of a 60-year-old male patient suffered from pancreatic cancer in example 20 after the treatment.

Figure 22 illustrates the MR image of a 56-year-old male patient suffered from thyroid cancer with trachea and lung metastasis in example 22 before the treatment.

Figure 23 illustrates the MR image of a 56-year-old male patient suffered from thyroid cancer with trachea and lung metastasis in example 22 before the treatment.

Figure 24 illustrates the MR image of a 52-year-old male patient suffered from moderately differentiated squamous cell laryngopharyngeal carcinoma in example 24 before the treatment.

### DESCRIPTION OF THE PREFERRED EMBODIMENTS

Preparation of Tumor Cells

### 1. Pre-purification Treatment of Tumor Cells

A piece of tumor tissue is excised and preserved in a culturing flask containing RPMI-1640 culture medium (serum-free). The necrotic tumor tissue, slough and non-tumor tissue are removed using a sterilized iris scissor. After being wet by a small amount of the RPMI-1640 culture medium (serum-free), the tumor tissue is cut into pieces, which are then placed in a 50-ml centrifuge tube. After adding about 10-15 ml of collagenase 4 solution (Sigma Co.) into the centrifuge tube, the tube is placed in a water bath at about 37°C for 1 hour. The collagenase 4 solution is formed by dissolving collagenase 4 in D-Hanks (HBSS solution that is free of calcium ions and magnesium ions) with a concentration of 1 mg/ml, followed by filtering and sterilization. After removing the centrifuge tube from the water bath, 50 ml of solution is removed from the centrifuge tube and is diluted one time using the RPMI-1640 culture medium. The cell suspension liquid is removed by a repeated drawing up of the solution. Using a sterilizer filter (e.g. 70 mesh), the cell suspension liquid is centrifuged for about 8 minutes at 1200 rpm/min. After centrifugation, the supernatant is removed, and 20 ml of 10% fetal calf serum or human AB serum and 1% double antibody RPMI-1640 culture medium are mixed with the cells (precipitate) to obtain a suspension of tumor cells.

### 2. Isolation and Purification of Tumor Cells

20 ml of deactivated fetal calf serum or normal human AB serum is placed in a 50-ml centrifuge tube labeled as A. 20 ml of a suspension evenly mixed cells from the tumor tissue (which includes lymphocytes, fibroblast, interstitial cells and necrotic cells) is gently added to the A-labeled centrifuge tube along the wall of the A-labeled centrifuge tube. The A-labeled centrifuge tube then remains undisturbed for about 6 minutes. Thereafter, 6 ml of the cell-serum solution is removed from the bottom of the A-labeled centrifuge tube and is placed in a B-labeled 50-ml centrifuge tube. The A-labeled centrifuge tube then remains undisturbed for 6 minutes. Thereafter, 6 ml of the cell-serum solution is removed from the bottom of the A-labeled centrifuge tube and is placed in a C-labeled 50-ml centrifuge tube. Then the A-labeled centrifuge tube remains undisturbed for 6 minutes. Hereafter, 6 ml of the cell-serum solution is removed from the bottom of the A-labeled centrifuge tube and is placed in a D-labeled 50-ml centrifuge tube. Thereafter, the B-labeled 50-ml centrifuge tube, C-labeled 50-ml centrifuge tube, D-labeled 50-ml centrifuge tube are being centrifuged at 1200 rpm for about 8 minutes. The resulting cells in the precipitate are purified tumor cells. 5 ml of a 10% fetal calf serum or human AB serum and 10 µg/ml penicillin, 50 µg/ml streptomycin, RPMI-1640 (serum free) culture medium are added to the cells in the precipitate in the B-labeled 50-ml centrifuge tube. After evenly mixed, the cell solution is placed in one hole of a 6-hole plate. The purified tumor cells can be observed through a microscope. After culturing the purified cells for about 3-4 days, cell divisions can be observed. The C-labeled 50-ml centrifuge tube and D-labeled 50-ml centrifuge tube contain a small amount of non-tumor cells asides from the tumor cells. The above process steps can be repeated to further purify the tumor cells.

### Preparation of Cellular Activator (Koyo) Derived from Tumor Cells Lystes

Fresh tumor cells are cut into pieces and filtered to form a suspension of mono-nuclear cells. Purified tumor cells are obtained after subjecting the suspension of cells through the isolation and purification process of the present invention. The 18234 purified tumor cells are inactive in liquid nitrogen repeatedly. The tumor cells are lysed and cellular activators (Koyo) are obtained. After being centrifuged, the supernatant is collected and filtered through a 0.22µm mesh for sterilization. After the protein content is detected, the cellular activators are preserved at -80°C.

### Preparation of Dendritic Cells Vaccine:

50 ml of the peripheral blood is retrieved from a patent for isolating the mono-nuclear cells. The mono-nuclear cells are isolated and preserved in a flask of culture medium and incubated at 37°C under 5% CO₂ for about 1 hour. The suspensive cells are then removed, and the remaining adherent cells are guided to differentiate into dendritic cells. After adding fresh dendritic cell culture medium (RPMI-1640 complete culture medium (5% human AB serum) + GM-CSF (100ng/ml) + IL-4 (30ng/ml)) to the adherent cells in the flask, the adherent cells are cultured in an incubator at 37°C under 5% CO₂. After two days of culturing, the autologous tumor cells lysate-derived cellular activators (Koyo) are added to the dendritic cells culture medium. On the sixth day, TNF-α is added and the mature DC vaccine is collected on the seventh day.

### Preparation of D-CIK cells proliferation

The suspensive cells are cultured in the RPMI-1640 medium (5% human AB serum + INFγ-1000U/ml) in an incubator at 37°C under 5% CO₂. After culturing for 24 hours, murine anti-human CD3 monoclonal antibody, 1000 U/ml IL-2 and 1000 U/ml IL-1α are added to a final concentration of 100mg/ml. Culturing is continued and observation is made every day. The culture medium is replaced every 2-3 days by adding fresh IL-2 culture medium. On the seventh day of culturing, the mature dendritic cells are mixed with the culture, and GM-CSF (final concentration: 800 to 1000 IU/ml), IL-2 (final concentration: 20 to 40 IU/ml), IL-7 (final concentration: 20 to 40 ng/ml) are also added. Around the fourteenth day of culturing, the D-CIK cells are collected, centrifuged, and rinsed. After rinsing, the D-CIK cells are suspended in 100 ml of saline solution and 5 ml of 20% human albumin and the resulting D-CIK cells suspension is infused back to the patient within 1 hour.

### Immunophenotypic analysis for mature DC cells and D-CIK cells

Staining is performed using FITC labeled murine anti-human CD80, CD83, CD86, MHC-1 AND MHC-11 monoclonal antibody and Flow Cytometry is used to detect the expression of the surface molecule on the surface antigen of the DC cells. Before the infusion back to the patent, a small amount of the D-CIK cells (0.5 to 1x10⁶) is collected and detected for lymophyte subsets distribution, mainly detecting the ratio of CD3+CD8+ and CD3+CD56+, using Cy-chrome^{™} anti-human CD3, Cy-chrome^{™} anti-human CD4, FITC anti-human CD8, PE anti-human CD19, PE anti-human CD56 (manufactured by Pharmingen Co).

According to the method of the present invention for activating dendritic cells, D-CIK cells (DC stimulated CIK cells) with cellular activator derived by the application of an isolation and purification technique on human tumor cells, the tumor cells that can be used include cells selected from the group consisting of parotid cancer, mediastinum transferred squamous cell carcinoma, melanoma, pancreatic cancer, thyroid cancer, duodenum papilloma cancer, prostate cancer, ovarian cancer, kidney cancer, lung cancer, breast cancer, nasopharyngeal carcinoma, cerebellum medulloblastoma, colon cancer, liver cancer, malignant glioma, rectal cancer, esophageal cancer, lung cancer.

Further, the method of the present invention for activating dendritic cells, D-CIK cells (DC stimulated CIK cells) with cellular activator derived by the application of an isolation and purification technique on human tumor cells can be used to prepare vaccine for improving symptoms of infections. The primary dendritic cells, the D-CIK cells can be obtained form umbilical cord blood or peripheral blood of the patient. The types of infections include but not limited to antigen, virus, bacteria, parasites, fungus.

According to the above vaccine for improving symptoms of viral infections of the present invention, the types of virus include but not limited to piconaviridae, caliciviridae, togaviridiae, flaviviridae, coronavirida, rhabdoviridae, filovirida, paramyxoviridae, orthomyxoviridae ; bunyaviridae, arenaviridae, reoviridae, retroviridae, hepadnaviridae, parvoviridae, papovaviridae, adenoviridae, herpersviridae and poxyviridae.

According to the above vaccine for improving symptoms of bacterial infections of the present invention, the bacteria are selected from the group consisting of P. aeruginosa, E. coli, Klebsiella species, Serratia, Pseudomonas, P. cepacia, Acinetobacter, S. epidermidis, E. facecalis, S. pneumoniae, S. aureus, Haemophilus, Neisseria, N. meningitides, Bacterioides, Citrobacter, Branhamella, Salmonella, Shigella species, S. pyrogenes species, Proteus species, Clostridium, Erysipelothrix species, Listeria species, Pasteurella multocida, Streptobacillus species, Spirillum species, Fusospirocheta species, Treponema palladium species, Borrelia species, Actinomycetes, Mycoplasma species, Chlamydia species, Rickettsia species, Spirochaeta, Legionella species, Mycobacteria species, Ureaplasma species, Streptomyces species, Trichomonas species and P. mirabilis.

According to the vaccine for improving symptoms of parasital infections of the present invention, the parasites are selected from the group consisting of Plasmodium falciparum, P. vicax, P. ovale, P. malaria, Toxoplasma gondii, Leishmania mexicana, L. tropica, L. major, L. aethiopica, L. donovanii, L. Trypanosoma cruzi, T. brucei, Schistosoma mansoni, S. haematobium, S. japonium, Trichinella spiralis, Wuchereria bancrofti, Brugia malayi, Entomoeba histolytica, Enterobius vermicularis, Taenia solium, T, saginata, Trichomonas vaginatis, T. hominis, T. tenax, Giardia lamblia, Cryptosporridium parvum, Pneumocytis carinii, Babesia bovis, B. divergens, B. microti, Isospora belli, Ascaris lumbricoides, necator americanis, Ancylostoma duodenale, Stronglyoides stercoralis, Capillaria philippinensis, Angiostrongylus cantonesis, Hymenolepis nana, Diphyllobothrium latum, Echinococcus granulosus, E. multilocularis, Paragoniumus westermani, Clonorchis sinensis, Ophisthorchisfelineus, O. viverrini, Fasciola hepatica, Sarcoptes scabiei, Pediculus humanus, Phthirus pubis and Dermatobia hominis.

According to the above vaccine for improving symptoms of fungus infections of the present invention, the fungus are selected from the group consisting of Cryptococcus neoformans, Blastomyces dermatitidis, Ajellomyces dermatitidis, Histoplasma capsulatum, Coccidioides immitis, Candida species, Aspergillus species, Rhizomucor species, Cunninghamella species, Apophysomyces species, Sporothrix schenkii, Paracoccidioides brasiliensis, Paseudallescheria boydii, Torulopsis glabrata, Dermatophytes species.

According to the above vaccine for improving symptoms of infections of the present invention, wherein the Candida species is elected from C. albicans, C. tropicalis, C. parapsilosis, C. guillermondii and C. krusei; the Aspergillus species is selected from A. fumigatus, A. flavus, and A. niger; and the Apophysomyces speces is selected from A. saksenaea, A. mucor and A. absidia.

According to the above vaccine for improving symptoms of antigen infections of the present invention, the antigen is selected from the group consisting of Vibrio cholerae, enterotoxigenic Escherichia coli, rotavirus, Clostridium difficle, Shigella, Salmonella typhi, Parainfluenza virus, influenza virus, Streptococcus mutans, Plasmodium falciparum, Staphyloccus aureus, rabies virus and Epstein-Barr virus.

According to the method of the present invention for activating dendritic cells, D-CIK cells (DC stimulated CIK cells) with cellular activator derived by the application of an isolation and purification technique on human tumor cells, vaccines can be prepared for improving symptoms of cancers and metastatic cancers. The primary dendritic cells and D-CIK cells are obtained from umbilical cord blood or peripheral blood of a patient. The vaccine is applicable for improving symptoms of epithelial cancers include but not limited to parotid cancer, mediastinum transferred squamous cell carcinoma, melanoma, pancreas cancer, thyroid gland papillomatous cystadenoma, duodenum papilloma cancer, prostate cancer, ovarian cancer, kidney cancer, lung cancer, breast cancer, nasopharyngeal carcinoma, cerebellum medulloblastoma, colon cancer, liver cancer, malignant glioma, rectal cancer, esophageal cancer, lung cancer, liver metastases from colon cancer and liver metastases from rectal cancer.

According to the method of the present invention for activating dendritic cells, D-CIK cells (DC stimulated CIK cells) with cellular activator derived by the application of an isolation and purification technique on human tumor cells, vaccines can be prepared for improving symptoms of auto-immune deficiency, wherein the primary dendritic cells, D-CIK cells are obtained from umbilical cord blood or periphery blood of a patient.

Example 1 : Purification of Human Kidney Cancer Cells

### A. Preparation of human kidney cancer cells suspension

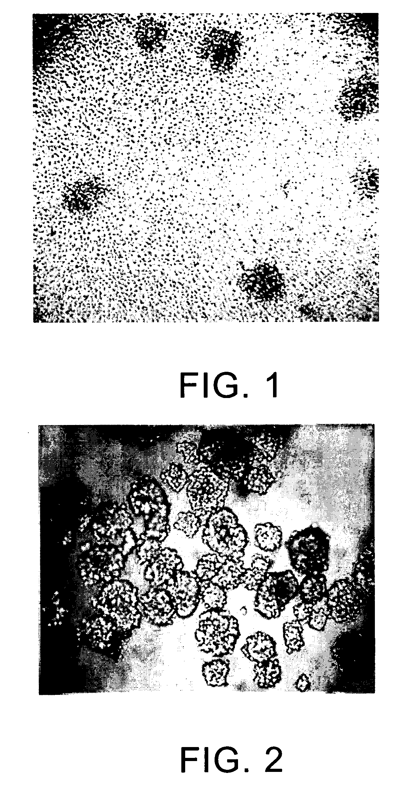

A piece of kidney cancer tissue (1-2 cm³) is excised and preserved in a culturing flask containing RPMI-1640 culture medium (serum-free). The necrotic tissue, slough and non-tumor tissue are removed using a sterilized iris scissor. After being wet by a small amount of the RPMI-1640 culture medium (serum-free), the tumor tissue is cut into pieces which are then placed in a 50-ml centrifuge tube. After adding about 10-15 ml of collagenase 4 solution (Sigma Co.) to the centrifuge tube, the centrifuge tube is placed in a water bath at about 37°C for 1 hour. The collagenase 4 solution is formed from dissolving collagenase 4 in D-Hanks (HBSS solution that is free of calcium ions and magnesium ions) with a concentration of 1 mg/ml, followed by filtering and sterilization. After removing the centrifuge tube from the water bath, the solution in the centrifuge tube is diluted one time using the RPMI-1640 culture medium, and the cell suspension liquid is removed by a repeated drawing up of the solution. Using a sterilizer filter (e.g. 70 mesh), the cell suspension liquid is centrifuged for about 8 minutes at 1200 rpm/min. After centrifugation, the supernatant is removed, and 20 ml of 10% fetal calf serum or human AB serum and 10 µg/ml penicillin and 50 µg/ml streptomycin RPMI-1640 culture medium are mixed with post-centrifuged cells (precipitate) to obtain a suspension of kidney cancer cells. Figure 1 illustrates a photograph of the kidney cancer cells observed under a microscope before purification.

### B. Isolation and Purification of Kidney Cancer Cells

### Isolation and Purification of Tumor Cells

20 ml of deactivated fetal calf serum or normal human AB serum is placed in a 50-ml centrifuge tube labeled as A. 20 ml of a suspension of the evenly mixed cells from the kidney cancer tissue (which include lymphocytes, fibroblast, interstitial cells and necrotic cells) (Figure 1) is gently added to the A-labeled centrifuge tube along the wall of the A-labeled centrifuge tube and remains undisturbed for about 6 minutes. Thereafter, 6 ml of the serum is removed from the bottom of the A-labeled centrifuge tube and placed in a B-labeled 50-ml centrifuge tube. The A-labeled centrifuge tube then remains undisturbed for 6 minutes. Thereafter, 6 ml of the serum is removed from the bottom of the A-labeled centrifuge tube and placed in a C-labeled 50-ml centrifuge tube. The A-labeled centrifuge tube then remains undisturbed for 6 minutes. Hereafter, 6 ml of the serum is removed from the bottom of the A centrifuge tube and placed in a D-labeled 50-ml centrifuge tube. Thereafter, the B-labeled 50-ml centrifuge tube, C-labeled 50-ml centrifuge tube, D-labeled 50-ml centrifuge tube are being centrifuged at 1200 rpm for about 8 minutes. The resulting precipitated cells are purified tumor cells. 5 ml of a 10% fetal calf serum or human AB serum and 10 µg/ml penicillin and 50 µg/ml streptomycin RPMI-1640 (serum free) culture medium are added to the precipitated cells in the B-labeled 50-ml centrifuge tube. After evenly mixed, some of the cell solution is placed in one hole of a 6-hole plate. It can be observed the strings of kidney cancer cells under a microscope (Figure 2). After culturing the purified cells for about 3-4 days, cell divisions can be observed. The C-labeled 50-ml centrifuge tube and D-labeled 50-ml centrifuge tube contain a small amount of non-tumor cells asides from the tumor cells. The above process steps can be repeated to further purify the tumor cells.

Example 2 : Kidney Patient

### Treatment Protocol:

All patients received DC vaccine immune therapy after 1-3 weeks of the clinic visits. DC vaccine was administered once per week for a total of at least eight times. The cell count of each treatment was greater than 1x10⁶. The DC vaccine was administered to the lymph notes at both sides of the groin and armpits via a subcutaneous injection. Regarding the D-CIK adoptive cells immune therapy, the CIK cells were cultured for 14-16 days. The cells were rinsed three times with saline solution and 10% of human albumin was added thereafter. The resulting D-CIK cells were infused to the patient with one injection. The patient received the D-CIK cells infusion one time per week for at least a total of 4 times. The cell count of each infusion was greater than 1x10¹⁰. Two days before each infusion of the D-CIK cells to the patient, test for bacteria was first conducted. The patient was allowed to continue the therapy according to the above protocols if requested even the required therapy was completed.

### Clinical Evaluation:

All clinical evaluations are conducted following the human tumor objective treatment effectiveness evaluation guidelines implemented by the Department of Health in 1988:
Complete response (CR): the tumor maintains disappeared for at least 4 weeks ;
Partial response (PR): the size of the tumor of the original nidus maintains being shrunk to 50% from the original maximum diameter for at least 4 weeks;
Stable disease (SD): the size of the tumor increases or diminishes less than 25% and no new nidus is identified;
Progressive disease (PD): the size of the tumor increases less than 25% or new nidus is identified;
For patient with no nidus to be evaluated, and image monitoring indicates no nidus is found, the evaluation will be rated as stable disease (SD); while image monitoring indicates metastasis or relapse will be rated as progressive disease (PD).

### Immune Status Evaluation

Ten patients are monitored for changes of T3(CD3+), T4(CD4+), T8(CD8+), CD4+/CD8+, NK cells (CD56+) in the peripheral blood before and after treatment. The monitoring is conducted once every two weeks. Delayed-type hypersensitivity (DTH) is also being monitored after each DC immune treatment. If a rash or a scleroma has a diameter greater than 5mm is developed, the result of the DHT test is marked as positive.

### Toxicity Monitoring:

Acute toxic reactions, such as hypersensitivity and abnormal reactions are being observed two hours after each treatment for the ten patients. Blood tests, liver function and kidney function evolutions are performed every other week to monitor chronic toxicity reaction.

### Statistics:

All numbers are presented in mean value with standard deviation using t-test for statistical analysis and P<0.05 for statistical significance.

The ten patients that are suffering from end-stage kidney cancer include seven males and three females. Their ages range between 19-70 years old with a mean age of 53 years old. The ECOG scores are 0-2. Five patients suffer from left kidney caner and the other five suffer from right kidney cancer. According to the biopsy, all ten cases are the clear cell type of kidney cancer.

Dendritic cells immune expression type: the cultured dendritic cells expresses the specific surface marker CD83 of the mature dendritic cells, MHC-I, MHC-II, category antigen, and also the co-stimulators including CD80 (B7-1) and CD86 (B7-2), etc. D-CIK cells surface analysis: the surface of the D-CIK cells include a group of heterogeneous cells, with CD3+CD4+ no greater than 15%, CD3+CD8+ no less than 60%, CD16+CD56+ no less than 10%, CD3+CD56+ no less than 15%.

Forty-eight hours before each DC and D-CIK treatment, bacteria tests are conducted on the DC and D-CIK cells to evaluate contamination. Once the contamination test is confirmed negative, the total DC cells are dissolved in 1.2 ml of normal saline and the resulting solution is subcutaneously injected to the patient at 3 immune spots. Each DC cell infusion requires a cell count of about 1.2~3.5x10⁶ cells/ml. Each D-CIK cell infusion requires a cell count of about 1.2~1.4x10¹⁰ cells/ml.

After the treatment, lymphocytes including CD3+, CD4+, the ratio of CD4+/CD8+ and CD56+ for all patients significantly increased (P<0.05). Regarding the test for DTH reaction, 6 patients have developed rash or scleroma that lasted for 2-5 days after 48 hours of the DC treatment.

Besides some minor discomforts such as low fever and chills have developed, no other serious reactions have been observed. Regular evaluations on the blood test, liver function and kidney function indicate no abnormal changes.

### Example 3 :

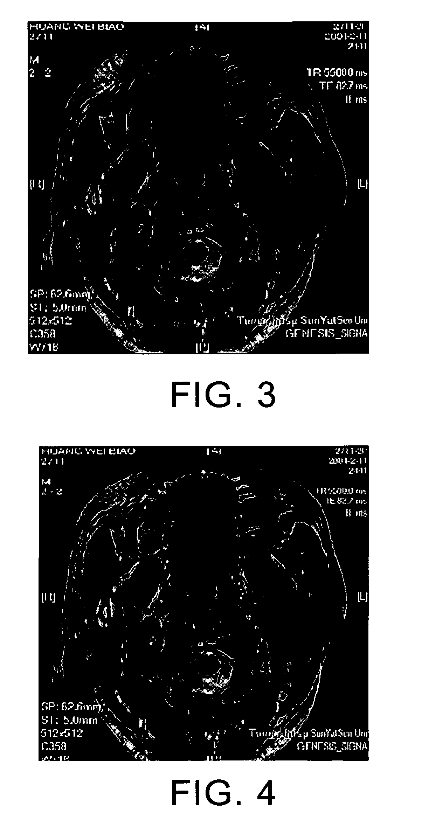

A 34-year-old male patient had developed a lump on the left earlobe in August of 2003 and a lump on the left temple in January of 2004. The patient was hospitalized on January 30th, 2004. The patient was diagnosed with left parotid cancer with low differentiation squamous cell carcinoma, T4N2M0, stage IVa. Parotidectomy on the were adopted on the right parotid and followed by chemotherapy :
Primary nidus 75Gy, 37 times, 58 days ;
Local lymph node : 50Gy, 25 times, 36days;
Chemotherapy: DDP+5-FU
Immune therapy: CIK infusion for 5 times 2 weeks after the chemotherapy. Currently, the patient is in stable condition. Figures 3 and 4 respectively illustrate the MR images before and after the treatment of the 34 years old male patient with parotid cancer.

### Example 4:

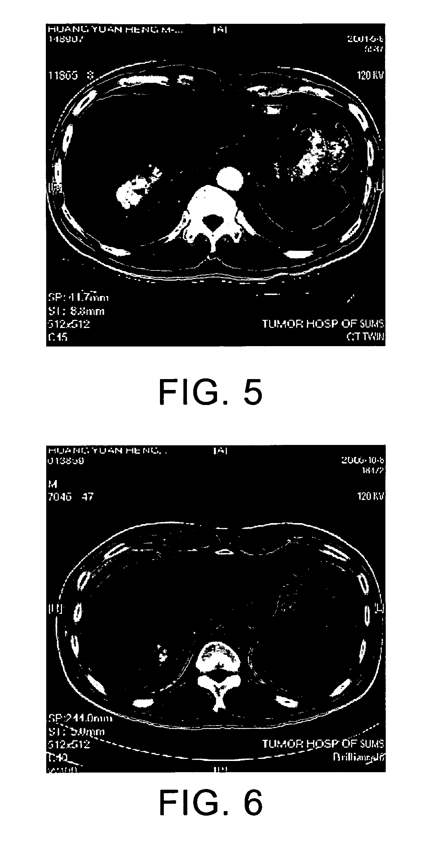

A 52-year-old male patient was hospitalized in 2004 due to complaints of upper gastrointestinal discomforts for about 2 months. The patient was diagnosed with liver cancer and treated by transcatheter arterial chemoembolization (liver TACE), radio frequency ablation (RFA), 125 Irradiation particle implant, and immune therapy by D-CIK infusion for 8 times. Currently, the patient is in stable condition and the cancer is under controlled. Figures 5 and 6 respectively illustrate the MR images before and after the treatment for the 52 years old male patient with liver cancer.

### Example 5:

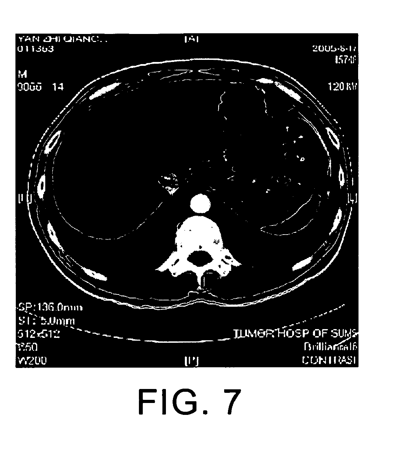

A 42-year-old male patient was hospitalized on August 16, 2005 due to the abnormal elevation of AFP. According to the computed tomography (CT) scan, patient was diagnosed with liver cancer on the left lobe. The patient received transcatheter arterial chemoembolization (liver TACE) on August 22^{nd}, 2005, radio frequency ablation (RFA) on September 1^{st}, 2005, D-CIK infusion through liver artery for eleven times during the period of September 16, 2005 to November 23, 2005. Currently, the patient is in stable condition. Figure 7 illustrates the MR images before the treatment for the 42 years old male patient with liver cancer.

### Example 6:

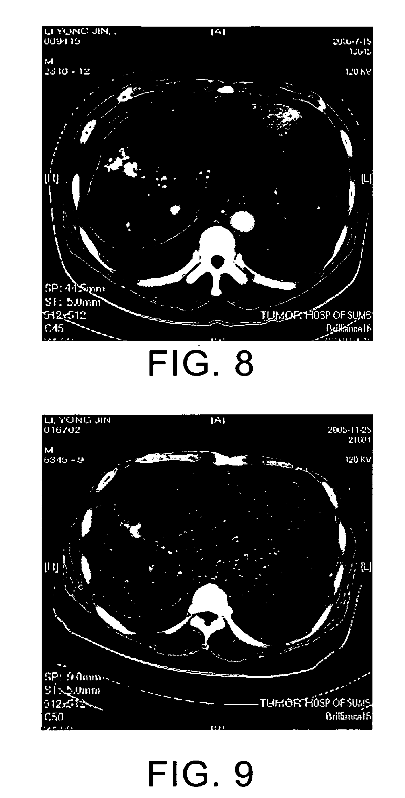

During a physical examination conducted in March 2003, intrahepatic lesion, which was suspected to be primary liver cancer, was found in a 55-year-old male patient with ultrasound. Total right lobectomy of the liver was performed in October 2003. According to the biopsy, patient was diagnosed with liver cancer grade III. Due the relapse after the surgery, percutaneous ethanol injection (PEI) was administered for three times, transcatheter arterial chemoembolization (liver TACE) for ten times. cThe CT scan conducted on November 21, 2005 indicated that the nidus was still present. Therefore, a CT-guided radio frequency ablation (RFA) was performed twice, followed by administering infusion of the D-CIK cells for 8 times. Currently, the patient is in stable condition. Figures 8 and 9 respectively illustrate the MR images before and after the treatment.

### Example 7 :

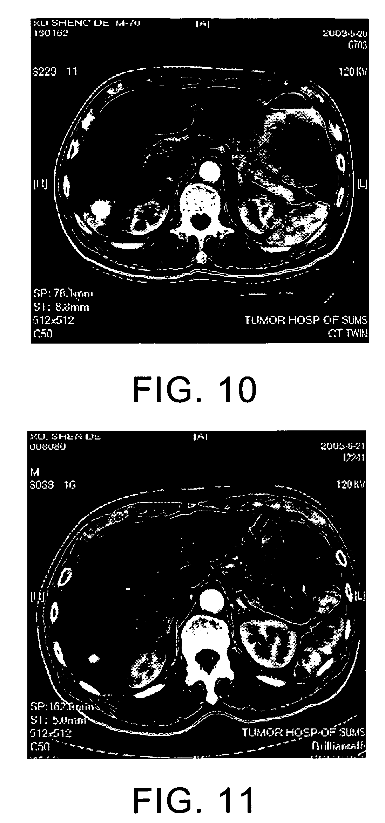

After the colon cancer surgery, intrahepatic lesions were found in a 72-year-old male patient. The patient was hospitalized on April 15, 2003. Radio frequency ablation (RFA) and percutaneous ethanol injection for the liver metastases were administered to treat the liver metasasis. Chemotherapy with Xloda (capecitabine) has also been conducted for one period of treatment.. Two weeks after the chemotherapy, immune therapy was conducted, in which D-CIK infusion was administered for 4 times. Currently, the patient is in a stable condition. Figures 10 and 11 respectively illustrate the MR images before and after the treatment.

### Example 8 :

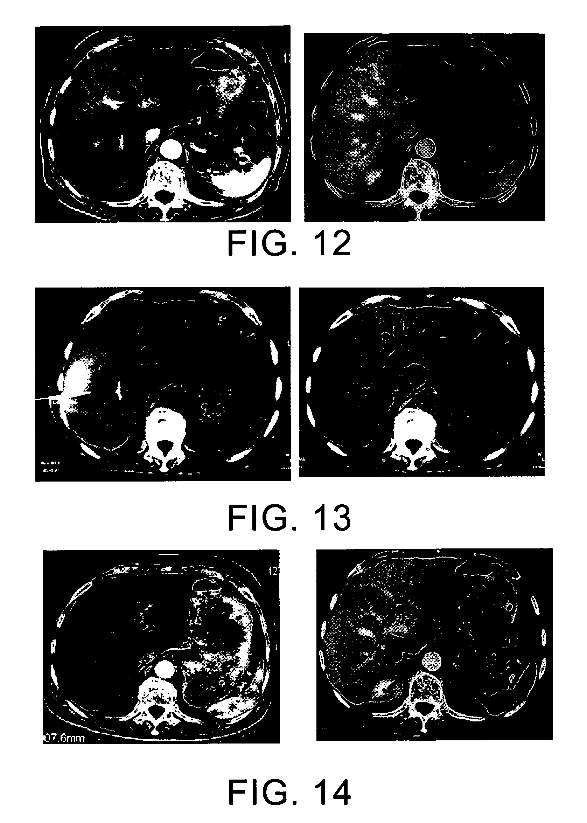

A 71-year-old female was patient was diagnosed with liver metastases from rectal cancer. The patient received a gastroendoscopic analysis on 6th May 2003 due to the constipation (pathology: tubular adnocarcinoma II grade). Based on the abdominal CT scan, the patient was diagnosed with liver metastases from rectal cancer. The patient received a rectal Dixon's operation on May 12^{th}, 2003, and followed by the radio frequency ablation (RFA) twice, the D-CIK cells infusion for 7 times, and the NK cells infusion for 4 times. Currently, the vital signs of the patient are stable, and the conditions of the patient are under controlled. Figures 12, 13 and 14 respectively illustrate the MR images of the patient before, during and after the treatment.

### Example 9 :

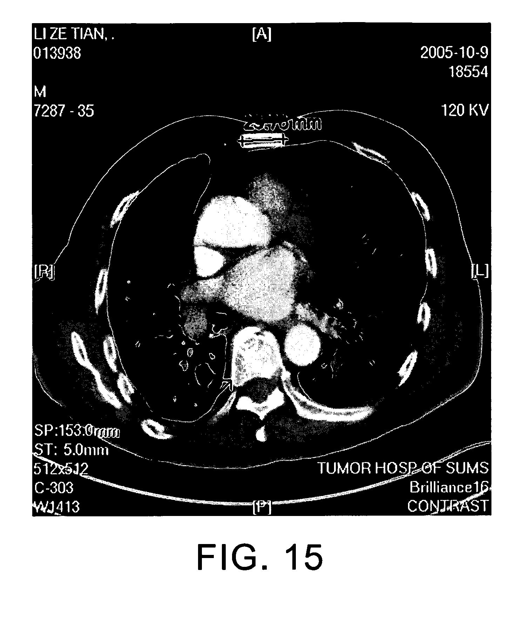

A lump was found at the lower right side of the lung of a 71-year-old male and the patient was hospitalized half a month later on November 4, 2004. The patient was diagnosed with lung adenocarcinoma at the lower right lobe, T1N0M0, stage I . After the resection of the lower right lobe of the lung and the mediastinal lymph node, and a wedge resection of the tip of the upper right lobe, pathology confirmed that the patient was suffering from lung adenocarcinoma at the right side, stage II, with metastasis extended to the pleura. Radiation particle implant of 125I was performed. Concurrently, immune therapy with D-CIK cells infusion was conducted for 8 times. Currently, the patient's condition is effectively under controlled. Figure 15 illustrates the MR images of the patient after the treatment.

### Example 10 :

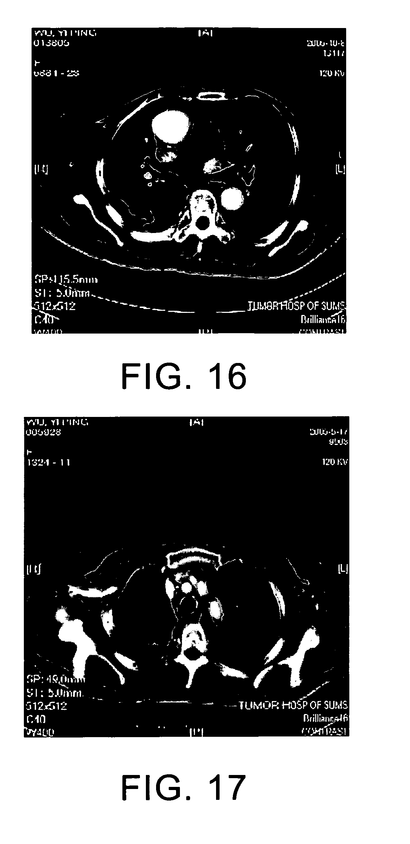

Pleural effusion at the right side of the chest and pleural thickening was discovered in a 63-year-old female patient during a physical examination. The patient was hospitalized a week later. Biopsy guided by CT scan confirmed the diagnosis that the patient had well differentiated adenocarcinoma at the right lung (T4N2M1). Chemotherapy using Taxol + Carboplatin was performed four times; and chemotherapy using Docetaxol+Carboplatin was performed twice. Three weeks after the chemotherapy treatment, D-CIK immune therapy was performed for 8 times. Currently, the patient is in stable condition. Figures 16 and 17 respectively illustrate the MR images of the patient before and after the treatment.

### Example 11 :

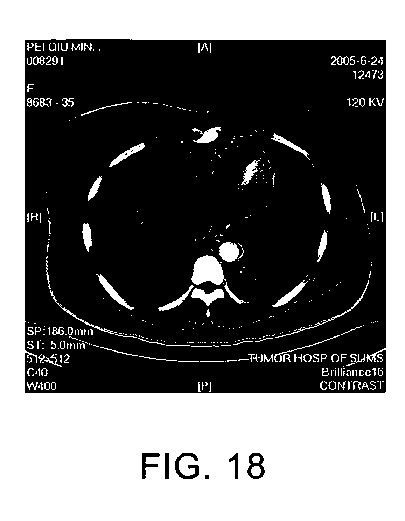

Intrahepatic lesion, which was suspected to be lung cancer, at the upper right lung was discovered in a 63-year-old male by chest CT scan on May 27th, 2005 after the patient has been suffering from dry cough. The tumor at the right lung was removed and lymphadenectomy was performed. Pathology results confirmed moderately differentiated adenocarcinoma, and partial bronchioloalveolar carcinoma. After surgery, chemotherapy (Gemcitabine + Carboplatin) was conducted for three times. Immune therapy was commenced on June 21st, 2005 and infusion of D-CIK cells was administered to the patient for 13 times. Currently, patient's condition is under controlled and the patient is in stable condition. Figure 18 illustrates the MR images of the patient after the treatment.

### Example 12 :

A 19 year-old-male patient was hospitalized on February 7^{th}, 2005 for nose bleeding for 6 months. Outpatient MRI indicated that the nasopharyngeal cavity was occupied by tumors, which confirmed nasopharyngeal non-keratinizing carcinoma (T3N1M0). Chemotherapy with PF solution was performed for 3 time, radio therapy was performed for multiple times (primary nidus: 68GY/30 times/38days; local lymph node: 64GY/30times/38days, lower neck for prevention: 50GY/25times/42 days). Thereafter, immune therapy was performed with IL-2+D-CIK infusion for 4 times. Currently, patient is under stable condition.

### Example 13:

A 60-year-old male patient received a CT scan due to complaints of dry cough and no improvement with anti-infection medication. Pathological changes in mediastinum was found and confirmed as mediastinum metastasis squamous cell carcinoma. After 3 periods of treatment of chemotherapy by NVB+DDP and one period of chemotherapy by Taxol+CBP, the nidus was shown to be reduced. In November of 2005, chemotherapy by Taxol + DDP was performed; and two weeks thereafter, immune therapy by D-CIK infusion was performed for 6 times. Currently, patient is in a stable condition.

### Example 14:

A 68-year-old male patient underwent chest X-ray and CT scan due to coughing. A lump on the left side of the lung and swelling of the mediastinum and the bronchopulmonary lymph nodes were discovered in the patent. The lung tumor needle biopsy confirmed the patient was suffering form adenocarcinoma grade II -III . Patient received 2 periods of chemotherapy started from 8th Oct., 2005, and on the third day after the completion of chemotherapy, the patient received radiation therapy. Chest examination that was subsequently performed confirmed that the size of the tumor had reduced. Chinese medicine and immune therapy were all administered at the same time. D-CIK infusion was performed for 12 times to effectively control the patient's condition.

### Example 15:

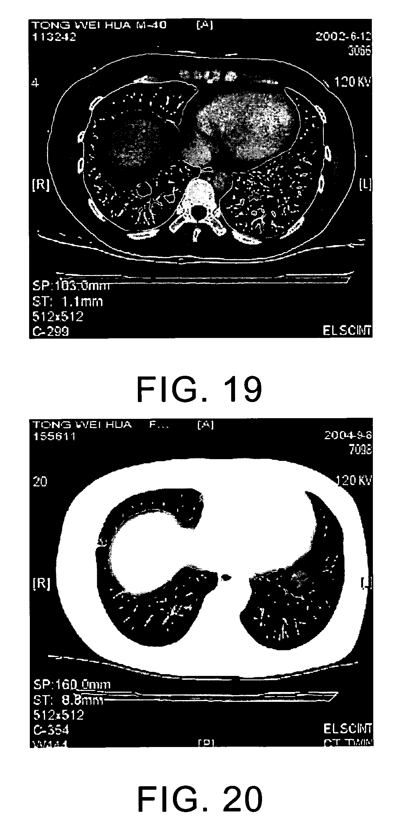

A 66-year-old male patient had complained about upper abdominal distension and discomfort, gastroesophageal reflux and eructation for over a year. Upper gastrointestinal endoscopic examination was performed on April 25^{th}, 2005, and the result confirmed gastric cancer. On April 30^{th}, 2005, the patient received a resection for gastric carcinoma. Pathology result indicated low differentiated adenocarcinoma. Immune therapy was started on August 25^{th}, 2005, in which D-CIK infusion was administered for 8 times. The patient did not receive chemotherapy or radiation therapy Currently, the patient's condition is under controlled and the patient is in stable condition. Figure 20 illustrates the MR images of the patient after the treatment.

### Example 16:

A 55-year-old male patient received a partial excision for duodenum papilloma carcinoma and received a Wipple's operation after a relapse in 2004. Through a CT scan conducted on September, 2005, metastases to the lung and retroperitoneal lymph nodes were observed. The patient received the 125I radiation particle implant for 4 times and immune therapy by D-CIK infusion for 4 times. Currently, patient is in stable condition.

### Example 17:

A 43-year-old female was discovered with lung metastasis, 3 years after receiving the colon cancer surgery. The patient received 2 years of chemotherapy The patient was again diagnosed with colon cancer. After the removal of the right half of the colon, the patient received chemotherapy by CF+5-Fu. However, after the discovery of lung metastasis, the chemotherapy regiment was changed to 5-Fu+Avastin. Two weeks after the chemotherapy, immune therapy by D-CIK infusion was administered for 6 times. The patient condition was under controlled and the nidus shrank.

### Example 18:

A 56-year-old female patient was hospitalized on November 14, 2004 after suffering from malignant melanoma at the left heal for three months. In March of 2004, the lump at the left heel was surgically removed. However, the wound healing was poor and a lump was again resurfaced. The lump on the left heel was surgically removed from the patient and skin implant was performed on August 10^{th}, 2005. Pathology result again confirmed malignant melanoma. On August 25^{th}, 2005 the patient started immune therapy of D-CIK+DC for 8 times. Currently, patient is stable condition. Figures 19 and 20 respectively illustrate the MR images of the patient before and after the treatment.

### Example 19:

A 52-year-old female patient was suspected of suffering from pelvic tumor due to ascites and a lump at the abdomen. Patient received a bilateral hysterectomy and a partial colectomy. Post-operative pathology result confirmed left fallopian tube carcinoma (acinar cell medullary carcinoma). After the surgical operation, chemotherapy was performed by TP solution for 2 cycles. Thereafter, pelvic and paraaortic lymphadenotomy and omentectomy and appendectomy were performed, followed by four cycles of chemotherapy by TP solution. In June of 2005, the tumor marker, CA125, of the patient was gradually elevated. According to the whole body positron emission tomography (PET)/computed tomography (CT) scans taken on November 25, 2005, the ascending colon liver region and lung metastases were found. Patient received whole body chemotherapy and radiation particle implant on December 2^{nd}, 2005. At the same time, the patient received immune therapy by D-CIK infusions for 4 times. Currently, patient's condition is stable.

### Example 20:

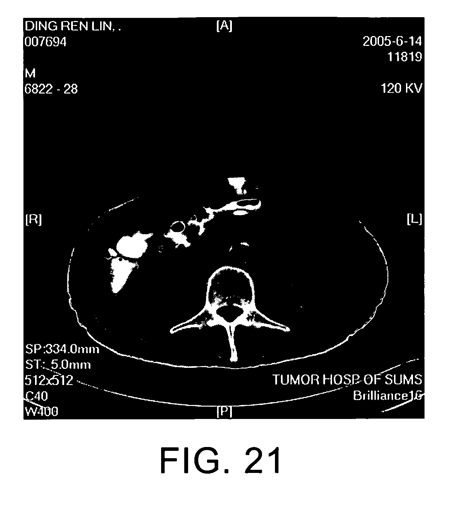

A lump with a size 4.7×3.0 cm² was found at the bottom part of the pancreas of a 60 year-old-male patient in June of 2003. The diagnosis was pancreatic cancer. Surgical operation was performed to remove the bottom part of pancreas and the spleen. Post-operative pathology result confirmed mucinous adenocarcinoma between moderately to low differentiation. Metastases of the Adipose tissue around the pancreas, nerve and lymph nodes were observed (1/3). Chemotherapy by 5-Fu abdominal pump was administered for 5 times, followed by radiation therapy for 28 times and chemotherapy by Gemcitabine for 8 times. However, relapse of pancreatic cancer and metastasis were discovered by PET scan. Patient was administered with Docetaxel - Gemcitabine - Iressa - C225, etc.. In June of 2005, the patient's GI function was damaged and the general condition of the patient was poor. Accordingly, anti-infection treatment and supportive care were provided, and immune therapy by IL-2+D-CIK was administered for 4 times. Patient's condition was under controlled. Figure 21 illustrates the MR images of the patient after the treatment.

### Example 21:

A lump was found at the right side of the kidney of a 38 years old male patient during a physical examination and the patient was hospitalized on December 24^{th}, 2004. Ultrasonic scan and CT scan confirmed the diagnosis of kidney cancer. The right side of kidney was resected and post-operative pathology result indicated the clear cell type of kidney cancer (well differentiated type) and small nidus with clear tumor cells around the abdominal aorta lymphatic tissues. Patient was administered with interferon, and immune therapy by D-CIK infusion was provided for 12 times to control the cancer. Currently, patient is in stable condition with no relapse.

### Example 22:

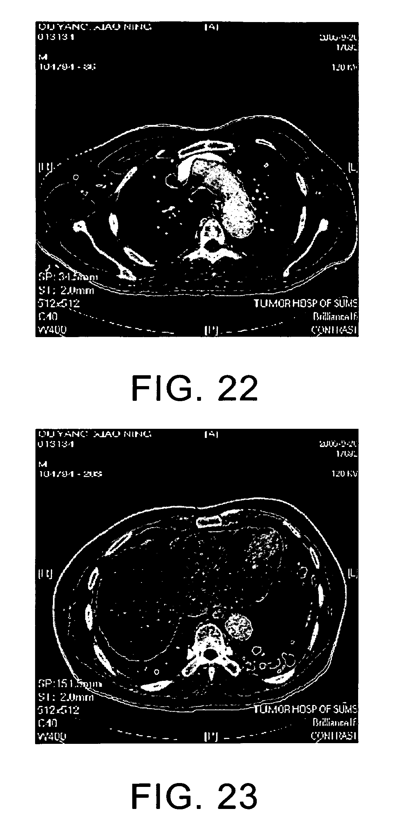

A 56-year-old male patient received an operation 3 years ago for thyroid cancer. According to the post-operative pathology result, the patient was diagnosed with right thyroid follicular papillary adenoma. No post-operative treatment was followed. Relapse was found with trachea and lung metastases in 2004. Bilateral total thyroidectomy and partial removal of the trachea were performed. A 131I treatment was administered for treating lung metastasis. Two other operations were performed to remove the neck lymph nodes due to metastasis. In August of 2005, the patient suffered from persistent swelling of the left shoulder, and the patient was hospitalized on September 22, 2005. According to MRI and bone ECT indicated overactive metabolic activities of the third thoracic vertebra, which suspected to be bone metastasis. Radiation therapy was administered, followed by immune therapy by D-CIK infusion for 4 times. Currently, patient is in stable condition. Figure 22 illustrates the MR image of the patient after the treatment.

### Example 23:

A 70-year-old male patient was hospitalized on October 23^{rd}, 2003 due to difficulty in passing urinate and PSA elevation. According to CT scan, prostatauxe with an overgrowth of prostate tissue pushed against the urethra and the bladder was observed. Pathology confirmed lower differentiated adenocarcinoma. Endocrine therapy was performed between October 23rd to November 3rd, 2003. Gemzer+DDP adjuvant chemotherapy of one cycle was performed on April 27th, 2004; combined therapy of gemcitabine andCisplatin Injection of one cycle was administered on May 19th, combined therapy of Gemzer + DDP of one cycle was administered on June 5th; and immune therapy by D-CIK infusion was administered for four times. The patient condition was effectively under controlled.

### Example 24:

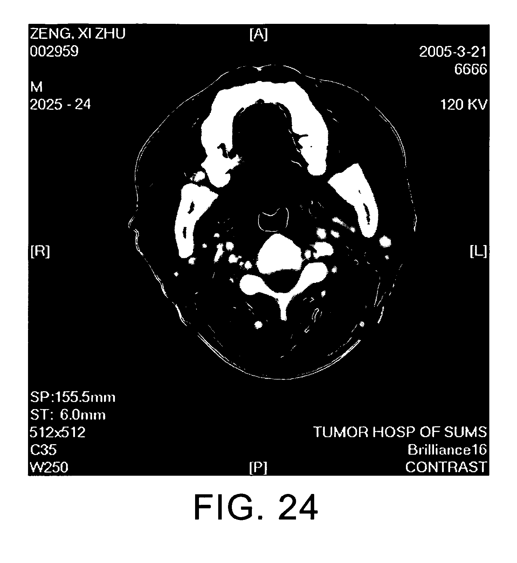

A 52-year-old male patient complained of dysphagia and blood in the sputum. Biopsy and flexible laryngoscope are performed and moderately differentiated squamous cell carcinoma was diagnosed. Between April 6^{th} and May 31^{st}, the patient received radiotherapy After the therapy, the tumor disappeared. On July 12^{th}, immune therapy by IL-2+D-CIK infusion for 5 times was performed. Currently, patient is in good condition. Figure 24 illustrates the MR images of the patient after the treatment

It will be apparent to those skilled in the art that various modifications and variations can be made to the structure of the present invention without departing from the scope or spirit of the invention. In view of the foregoing descriptions, it is intended that the present invention covers modifications and variations of this invention if they fall within the scope of the following claims and their equivalents

## Claims

1. A method for purifying and isolating human tumor cells, wherein cellular activators are derived from purified tumor cells, the method comprising:
performing a pre-purification procedure to obtain a suspension of cells from a tumor tissue; and
performing an isolation and purification procedure comprising:
A. adding a serum to the suspension of cells to form a cell-serum solution in a container;
B. allowing the cell-serum solution to remain undisturbed for a period of time;
C. collecting a portion of the cell-serum solution from a bottom of the container; and
D. centrifuging the portion of the cell-serum solution collected from the bottom of the container to obtain the purified tumor cells in a precipitate of the post-centrifuged cell-serum solution.

2. The method of claim 1, wherein the steps B to C are repeated.

3. The method of claim 1, wherein the serum comprises a deactivated fetal calf serum or a normal human AB serum.

4. The method of claim 1, the cell-serum solution remains undisturbed for about 6 minutes.

5. The method of claim 1, wherein the portion of the cell-serum solution is centrifuged at 1200 rpm for about 8 minutes.

6. The method of claim 1, wherein the pre-purification procedure to obtain the suspension of cells comprises:
removing non-tumor tissues and sloughs from the tumor tissue;
rinsing the tumor tissue with a serum-free culture medium;
cutting the tumor tissue into small pieces;
adding a collagenase solution to the small pieces of the tumor tissue;
incubating the collagenase solution with the tumor tissue in a water bath;
diluting the collagenase solution with the tumor tissue one time with the serum-free culture medium;
centrifuging the collagenase solution with the tumor tissue;
removing a supernatant from the post-centrifuged collagenase solution with the tumor tissue; and
adding the serum and the culture medium to a precipitate of the post-centrifuged collagenase solution with the tumor cells to form the suspension of the cells.

7. The method of claim 5, wherein the collagenase solution with the tumor tissue is incubated at 37°C for about one hour.

8. The method of claim 1, wherein deriving the cellular activators from the purified tumor cells comprises:
lysing the purified tumor cells;
centrifuging the lysed purified tumor cells; and
collecting the cellular activators from a supernatant of the centrifuged lysed purified tumor cells solution.

9. The method of claim 1, wherein the human tumor cells are selected from the group consisting of parotid cancer, mediastinum transferred squamous cell carcinoma, melanoma, pancreatic cancer, thyroid cancer, duodenum papilloma cancer, prostate cancer, ovarian cancer, kidney cancer, lung cancer, breast cancer, nasopharyngeal carcinoma, cerebellum medulloblastoma, colon cancer, liver cancer, malignant glioma, rectal cancer, esophageal cancer, lung cancer.

10. A method for activating dendritic cells (DC) by the cellular activators derived from the cellular activators obtained according to the method of claim 8, wherein the method for activating dendritic cells (DC) by the cellular activators comprises:
isolating mono-nuclear cells from a blood sample;
incubating the mono-nuclear cells;
removing a cell suspension to collect adherent cells;
culturing the adherent cells in a culture medium; and
adding the cellular activators (Koyo) to the adherent cells after two days of culturing; and
collecting mature dendritic cells.

11. The method of claim 10, wherein the blood sample is retrieved from umbilical cord blood or periphery blood.

12. A method for preparing D-CIK cells, the method comprising:
culturing suspsensive cells in a RPMI-1640 culturing medium at 37°C and under 5%CO₂;
adding murine anti-human CD3 monoclonal antibody, (interleukin) IL-2, IL-1 to the suspensive cells in the culturing medium;
adding mauture dendritic cells, GM-CSF (Granulocyte-macrophage colony-stimulating factor), IL-2 and IL-7 to the culture; and
collecting the D-CIK cells.

13. The method of claim 12, wherein the D-CIK cells are infused to a patient within one hour the D-CIK cells are prepared.

14. An application of the cellular activator "Koyo", derived from the purified tumor cells purified and isolated according to the method of claim 1, to activate dendritic cells or D-CIK cells for a preparation of a vaccine to improve infections.

15. The application of the cellular activator "Koyo" of claim 14, wherein types of the infections are selected from the group consisting of antigen, virus, bacteria, parasites and fungus.

16. The application of the cellular activator "Koyo" of claim 15, wherein types of virus are selected from the group consisting of piconaviridae, caliciviridae, togaviridiae, flaviviridae, coronavirida, rhabdoviridae, filovirida, paramyxoviridae, orthomyxoviridae ; bunyaviridae, arenaviridae, reoviridae, retroviridae, hepadnaviridae, parvoviridae, papovaviridae, adenoviridae, herpersviridae and poxyviridae.

17. The application of the cellular activator "Koyo" of claim 15, wherein types of bacteria are selected from the group consisting of P. aeruginosa, E. coli, Klebsiella species, Serratia, Pseudomonas, P. cepacia, Acinetobacter, S. epidermidis, E. facecalis, S. pneumoniae, S. aureus, Haemophilus, Neisseria, N. meningitides, Bacterioides, Citrobacter, Branhamella, Salmonella, Shigella species, S. pyrogenes species, Proteus species, Clostridium, Erysipelothrix species, Listeria species, Pasteurella multocida, Streptobacillus species, Spirillum species, Fusospirocheta species, Treponema palladium species, Borrelia species, Actinomycetes, Mycoplasma species, Chlamydia species, Rickettsia species, Spirochaeta, Legionella species, Mycobacteria species, Ureaplasma species, Streptomyces species, Trichomonas species and P. mirabilis.

18. The application of the cellular activator "Koyo" of claim 15, wherein types of parasites are selected from the group consisting of Plasmodium falciparum, P. vicax, P. ovale, P. malaria, Toxoplasma gondii, Leishmania mexicana, L. tropica, L. major, L. aethiopica, L. donovanii, L. Trypanosoma cruzi, T. brucei, Schistosoma mansoni, S. haematobium, S. japonium, Trichinella spiralis, Wuchereria bancrofti, Brugia malayi, Entomoeba histolytica, Enterobius vermicularis, Taenia solium, T, saginata, Trichomonas vaginatis, T. hominis, T. tenax, Giardia lamblia, Cryptosporridium parvum, Pneumocytis carinii, Babesia bovis, B. divergens, B. microti, Isospora belli, Ascaris lumbricoides, necator americanis, Ancylostoma duodenale, Stronglyoides stercoralis, Capillaria philippinensis, Angiostrongylus cantonesis, Hymenolepis nana, Diphyllobothrium latum, Echinococcus granulosus, E. multilocularis, Paragoniumus westermani, Clonorchis sinensis, Ophisthorchisfelineus, O. viverrini, Fasciola hepatica, Sarcoptes scabiei, Pediculus humanus, Phthirus pubis and Dermatobia hominis.

19. The application of the cellular activator "Koyo" of claim 15, wherein types of fungus are selected from the group consisting of Cryptococcus neoformans, Blastomyces dermatitidis, Ajellomyces dermatitidis, Histoplasma capsulatum, Coccidioides immitis, Candida species, Aspergillus species, Rhizomucor species, Cunninghamella species, Apophysomyces species, Sporothrix schenkii, Paracoccidioides brasiliensis, Paseudallescheria boydii, Torulopsis glabrata, Dermatophytes species.

20. The application of the cellular activator "Koyo" of claim 15, wherein types of antigen are selected from the group consisting of Vibrio cholerae, enterotoxigenic Escherichia coli, rotavirus, Clostridium difficle, Shigella, Salmonella typhi, Parainfluenza virus, influenza virus, Streptococcus mutans, Plasmodium falciparum, Staphyloccus aureus, rabies virus and Epstein-Barr virus.

21. An application of the cellular activator "Koyo", derived from the purified tumor cells purified and isolated according to the method of claim 1, to activate dendritic cells or D-CIK cells for a preparation of a vaccine to improve auto-immune deficiency.

22. An application of the cellular activator "Koyo" derived from the purified tumor cells purified and isolated according to the method of claim 1 to activate dendritic cells or D-CIK cells for a preparation of a vaccine to improve symptoms of cancers and metastatic cancers.

23. The application of the cellular activator "Koyo" of claim 22, wherein types of the cancers are selected from the group consisting of parotid cancer, mediastinum transferred squamous cell carcinoma, melanoma, pancreas cancer, thyroid gland papillomatous cystadenoma, duodenum papilloma cancer, prostate cancer, ovarian cancer, kidney cancer, lung cancer, breast cancer, nasopharyngeal carcinoma, cerebellum medulloblastoma, colon cancer, liver cancer, malignant glioma, rectal cancer, esophageal cancer, lung cancer, liver metastases from colon cancer and liver metastases from rectal cancer.
